# FASCICULE DE BREVET EUROPEEN

(11) **EP 0 690 747 B1**
(45) Date de publication et mention de la délivrance du brevet: **28.05.1997**
(21) Numéro de dépôt: 94909968.3
(22) Date de dépôt: 15.03.1994
(51) Int. Cl.: B01J 13/04

(54) **PROCEDE DE PREPARATION DE PARTICULES RENFERMANT UN INGREDIENT ACTIF PAR EXTRUSION ET LYOPHILISATION**
VERFAHREN ZUR HERSTELLUNG VON WIRKSTOFF ENTHALTENDEN TEILCHEN DURCH EXTRUSION UND LYOPHILISATION
EXTRUSION AND FREEZE-DRYING METHOD FOR PREPARING PARTICLES CONTAINING AN ACTIVE INGREDIENT

(30) Priorité: 23.03.1993 FR 9303316
(43) Date de publication de la demande: 10.01.1996
(73) Titulaire: LABORATOIRE L. LAFON, 94700 Maisons Alfort Cédex (FR)
(72) Inventeur: NGUYEN, Thanh-Tam, F-94450 Limeil-Brevannes (FR); JACQUOT-LEYDER, Jöelle, F-94000 Créteil (FR)
(74) Mandataire: Clisci, Serge
(86) Numéro de dépôt international: FR9400281
(87) Numéro de publication internationale: WO9421371

(56) Documents cités:
- EP-A- 0 204 596
- EP-A- 0 438 359
- GB-A- 2 004 182
- GB-A- 2 133 983
- US-A- 4 230 687

## Description

La présente invention a trait à un nouveau procédé de préparation de particules isolées, qui renferment chacune au moins un ingrédient actif utile en thérapeutique, cosmétique, diététique ou alimentation, par extrusion puis lyophilisation.

Elle concerne également en tant que produits industriels nouveaux lesdites particules constituées par une association intime d'un excipient physiologiquement acceptable et d'au moins un ingrédient actif et obtenues selon ledit procédé par extrusion puis lyophilisation.

Ces particules, qui sont dénommées ci-après "microparticules" et qui ont une dimension maximale comprise entre 0,05 mm et 5 mm, sont obtenues sensiblement sous la forme de bâtonnets ou, mieux, sous la forme de sphères (également dénommées "microsphères", "perles", "billes" ou "microbilles").

### ART ANTERIEUR

On sait que l'on a déjà obtenu pour usage thérapeutique ou alimentaire des microparticules par (i) extrusion, à une température en général supérieure ou égale à 45°C, d'un mélange intime d'un ingrédient actif et d'un excipient physiologiquement acceptable fusible, à travers une tête d'extrusion comportant une ou plusieurs filières, (ii) découpe, au niveau de chaque filière, de l'extrudat résultant notamment au moyen d' une lame ou au moyen de vibrations périodiques, et (iii) séchage des particules résultantes, tombant en général par gravité, au moyen d'un gaz inerte ascendant (i.e. circulant à contre-courant du trajet des particules). Voir à cet effet la demande de brevet européen publiée EP-A-0204596 qui décrit l'obtention de bâtonnets cylindriques (cf. colonne 4 lignes 52-57), d'une part, et la demande de brevet européen publiée EP-A-0 438 359 et la demande de brevet allemande publiée DE-B-2 725 924 ("Auslegeschrift") qui décrivent l'obtention de microparticules sphériques par extrusion sous vibrations (notamment d'une fréquence de 200-400 Hz ou de 1800-2500 Hz selon DE-B 2 725 924), d'autre part. D'autres modalités de la technique d'extrusion figurent dans le brevet américain délivré US-A-2 918 411 et la demande de brevet européen publiée EP-A-0 465 338.

Selon l'art antérieur précité, l'excipient comprend ou est essentiellement constitué par un matériau lipidique, de préférence non hydrosoluble, qui est fusible et qui va intervenir en tant que solvant de l'ingrédient actif. Ce matériau lipidique est requis pour obtenir des microparticules de forme régulière notamment des microbilles, qui n'adhèrent ni ne s'agglomèrent entre elles au cours de la solidification. Selon ledit art antérieur, on chauffe ledit matériau lipidique pour le fondre, introduit dans la masse fondue résultante l'ingrédient actif et, le cas échéant, les autres composants de l'excipient physiologiquement acceptable pour former un mélange ayant une viscosité suffisante [inférieure à 60 cP (i.e. 0,06 Pa.s) et de préférence comprise entre 10 et 20 cP (i.e. 0,01 et 0,02 Pa.s) ; selon les indications fournies dans DE-B-2 725 924 colonne 3, lignes 52-57] à la température de la tête d'extrusion; et, procède à la découpe de l'extrudat (à la sortie de la tête d'extrusion) au moyen d'une lame ou couteau (obtention de bâtonnets) ou de vibrations périodiques (obtention de microsphères) pour former des gouttelettes que l'on solidifie, en général, au moyen d'un contre-courant de gaz inerte tandis qu'elles tombent par gravité.

En particulier, EP-A-0 438 359 rappelle (voir page 2, lignes 25-29) que l'utilisation d'un contre-courant ou jet gazeux très froid (-10°C à -20°C) sur le courant de microparticules présente l'inconvénient d'augmenter la viscosité du produit que l'on veut solidifier et par suite de ralentir la cristallisation de l'ingrédient actif. Aussi, EP-A-0 438 359 préconise la mise en oeuvre d'un refroidissement qui n'est pas aussi intense (voir page 2 lignes 30-31) et l'utilisation d'un lit fluidisé au niveau du réceptacle des microparticules pour maintenir en fluidisation les microsphères non encore entièrement solidifiées.

Il convient également de remarquer que US-A-2 918 411 prévoit la solidification à la température ambiante de la masse fondue (ce brevet utilise l'expression "congealed mass" colonne 2, ligne 28 où "congealed" a le sens de "solidifié" ainsi que cela ressort des modalités opératoires données à l'exemple 1 ; voir à cet effet, colonne 3, lignes 67-68, où figure l'expression : "the congealed mass is cooled to room temperature").

L'utilisation d'une masse pâteuse contenant de l'eau va manifestement à l'encontre de l'enseignement de l'art antérieur relatif à la technique d'extrusion et illustré par les documents DE-B-2 725 924, US-A-2 918 411, EP-A-0 438 359, EP-A-0 465 338 et EP-A-0 204 596 précités.

On sait que la lyophilisation est un cas particulier de la cryodessiccation, lorsque le solvant d'un matériau que l'on veut éliminer est l'eau. La lyophilisation comprend une phase dite de congélation puis une phase dite de sublimation au cours de laquelle l'eau que l'on veut éliminer est chassée par réchauffement sous pression réduite. La technique de lyophilisation est bien connue et peut être notamment illustrée par les brevets EP-B-0 159 237, US-A-4 178 695, US-A-4 490 407 et US-A-4 883 507.

Les brevets US-A-4 490 407 et US-A-4 883 507 décrivent en particulier l'obtention de microparticules, enrobées et sphériques, ayant un diamètre compris dans l'intervalle de 10-120 µm (selon US-A-4 490 407) ou de préférence inférieur à 1 µm (selon US-A-4 883 507), par nébulisation, séchage au moyen d'un courant gazeux puis (a) lyophilisation dans des alvéoles d'une composition aqueuse contenant lesdites microparticules enrobées (voir US-A-4 490 407 colonne 3, lignes 1-3 et colonne 4, lignes 32-33) ou (b) recueil sur un filtre constitué d'une masse poreuse aux gaz et obtenue par congélation, broyage, sublimation du solvant et compression (voir US-A-4 883 507 de la colonne 10 ligne 30 à la colonne 11 ligne 26).

On sait par ailleurs que la lyophilisation présente un certain nombre d'avantages. Elle permet de conserver les caractéristiques initiales des ingrédients actifs ou celles qui ont été élaborées au cours de leur fabrication, d'une part, et de protéger les ingrédients actifs des altérations dues à la chaleur et à l'eau (en évitant notamment les réactions d'hydrolyse et d'oxydation), d'autre part.

Elle améliore lors du stockage la stabilité des ingrédients actifs, en particulier à deux niveaux :
- la stabilité chimique qui évite l'altération des molécules présentes sous forme de fines particules actives, et
- la stabilité physique qui évite la dénaturation des caractéristiques de la forme obtenue, ladite dénaturation entraînant un ralentissement de la libération de l'ingrédient actif et une modification des propriétés organoleptiques (telles que la consistance et le goût), un tel inconvénient constituant une gêne en ce qui concerne l'observance (i.e. le suivi et le respect de la posologie prescrite) et par suite l'efficacité de l'ingrédient actif.

La lyophilisation permet aussi d'éviter les transformations physiques des produits secs après le départ du solvant (ici l'eau), telles que les recristallisations et polymorphismes comme cela est souvent le cas dans une évaporation liquide/vapeur par la chaleur, d'une part, et d'obtenir à partir de substances à dissolution lente, des préparations plus facilement solubles dans l'eau, d'autre part. Ainsi lors de la phase de sublimation, qui intervient dans la lyophilisation pour éliminer l'eau, les molécules dissoutes ne s'agglomèrent pas pour former des cristaux, comme cela est le cas lors de l'évaporation, et le produit sec obtenu reste en théorie aussi divisé qu'il l'était dans la solution initiale.

La lyophilisation permet en outre l'association de substances incompatibles sur le plan physico-chimique en solution. De ce point de vue, elle permet le remplacement de compositions effervescentes, notamment les comprimés effervescents, par des lyophilisats.

Enfin, la lyophilisation contribue au traitement de surface des particules pour augmenter leur caractère hydrophile. Ainsi, les lyophilisats oraux à base d'ingrédients actifs, normalement insolubles ou peu solubles dans l'eau, donnent dans l'eau une suspension dans laquelle ils se retrouvent dans l'état conféré initialement à la suite de traitements tels que micronisation, dispersion, traitements de surface, etc. De plus, la structure poreuse des lyophilisats empêche l'agglomération des particules lors d'une dispersion desdits lyophilisats dans l'eau : l'intégrité de la granulométrie de départ est respectée et les phénomènes électrostatiques particulièrement gênants sont éliminés.

### BUT DE L'INVENTION

Il existe un besoin d'améliorer la biodisponibilité des ingrédients actifs, qui sont conditionnés sous une forme géométrique régulière du type matrice d'excipient physiologiquement acceptable renfermant dans sa masse lesdits ingrédients actifs et qui est obtenue par extrusion.

Il existe également un besoin de fournir des particules matricielles du type précité qui présentent les avantages des lyophilisats.

Ainsi suivant l' invention, on se propose de fournir une nouvelle solution technique mettant en oeuvre une extrusion et une lyophilisation, pour satisfaire les besoins précités et obtenir des particules de forme géométrique régulière présentant les avantages conférés par la lyophilisation. Cette nouvelle solution technique, qui comprend l'extrusion d'un mélange pâteux contenant de l'eau, va à l'encontre de l'enseignement de l'art antérieur en ce qui concerne les modalités d'extrusion, suivant lesquelles il fallait (i) éviter la présence d'eau dans le matériau que l'on voulait extruder, et (ii) faire appel en conséquence à un matériau lipidique fusible dans lequel l'ingrédient actif a été solubilisé, pour obtenir après solidification des particules de forme géométrique régulière.

Selon un premier aspect de l'invention, l'on se propose de fournir un procédé de préparation de particules isolées et géométriquement régulières, chacune du type matrice d'excipient renfermant dans sa masse au moins un ingrédient actif, ledit procédé évitant l'agglomération desdites particules entre elles ou avec les parois de leur réceptacle au cours de leur formation.

Selon un second aspect de l'invention, l'on se propose de fournir des particules obtenues selon ce procédé, à savoir par extrusion d'un mélange pâteux contenant de l'eau puis lyophilisation, lesdites particules renfermant chacune au moins un ingrédient thérapeutiquement, cosmétiquement, diététiquement ou alimentairement actif qui est utile tant chez l'homme que chez l'animal.

Selon un troisième aspect de l'invention, l'on se propose de fournir un procédé de conditionnement suivant lequel on revêt chacune desdites particules d'un enrobage polymère à paroi continue. La technique d'enrobage utilisée suivant l'invention est, comme on le verra plus loin, différente de celle de l'enrobage des comprimés non applicable ici eu égard aux dimensions et surtout à la porosité des lyophilisats.

### OBJET DE L'INVENTION

Le but de l'invention est atteint grâce à une nouvelle solution technique pour la préparation de particules matricielles par extrusion ou formage puis lyophilisation.

L'on préconise suivant l'invention un procédé pour la préparation de particules utiles notamment en thérapeutique, chaque particule comprenant un excipient formant une matrice et au moins un ingrédient actif régulièrement réparti dans la masse de la matrice, ledit procédé étant caractérisé en ce qu'il comprend la réalisation de noyaux secs de forme régulière, de préférence sphérique par extrusion ou formage puis lyophilisation, chaque noyau sec étant ensuite susceptible d'être enrobé, d'une part, et susceptible d'intervenir dans une préparation plus complexe, d'autre part.

Ce procédé comprend, selon une variante de mise en oeuvre, plus particulièrement
- la préparation d'un mélange pâteux ayant une viscosité, mesurée à la température ambiante (15-20°C), inférieure à 1 Pa.s,
- l'extrusion dudit mélange pâteux et la découpe de l'extrudat, ainsi obtenu, sous forme de corpuscules humides d'une taille généralement comprise entre 0,01 et 5 mm,
- la congélation desdits corpuscules par contact avec un fluide inerte à une température inférieure à 0°C, puis
- le séchage desdits corpuscules, ainsi congelés, par cryodessiccation.

La congélation est réalisée au cours de la chute des corpuscules humides dans un fluide gazeux refroidi circulant de préférence à contre-courant.

L'on préconise également suivant l'invention, en tant que produit industriel les particules, éventuellement enrobées à l'intérieur d'une membrane polymère à paroi continue, qui ont été obtenues selon ledit procédé et qui ont une dimension maximale comprise entre 0,05 et 5 mm.

### BREVE DESCRIPTION DES DESSINS

Les dessins joints en annexe, ne sont pas limitatifs mais illustrent d'autres avantages et caractéristiques de l'invention.
Plus précisément :
- la figure 1 représente schématiquement une installation permettant de mettre en oeuvre le procédé de préparation de l'invention à l'échelle industrielle ;
- la figure 2 représente sous forme de diagramme un mode de mise en oeuvre du procédé de l'invention ; et
- la figure 3 représente schématiquement une particule suivant l'invention (ici une microbille) obtenue par extrusion, lyophilisation puis enrobage.

### DESCRIPTION DETAILLEE DE L'INVENTION

Le procédé suivant l'invention permet d'obtenir des particules (dites ici "microparticules") de forme géométrique régulière du type matrice d'excipient contenant dans sa masse au moins un ingrédient actif.

Le procédé selon l'invention pour la préparation de particules comprenant chacune un excipient formant une matrice et au moins un ingrédient actif régulièrement réparti dans la masse de ladite matrice, qui comprend les opérations d'extrusion puis de lyophilisation, est caractérisé en ce qu'il comprend les étapes consistant dans :
(1°) la préparation d'un mélange homogène à partir
   (a) d'au moins un ingrédient actif,
   (b) d'un excipient hydrophile physiologiquement acceptable, et
   (c) d'eau,
   de façon à obtenir un mélange pâteux ayant une viscosité, mesurée à la température ambiante (15-20°C), inférieure à 1 Pa.s ;
(2°) l'extrusion du mélange homogène, ainsi obtenu, et la découpe de l'extrudat pour obtenir des corpuscules humides ;
(3°) la congélation desdits corpuscules, ainsi obtenus, pendant qu'ils tombent par gravité dans un courant gazeux inerte ayant une température inférieure à 0°C ; et,
(4°) le séchage desdits corpuscules par cryodessiccation.

Le cas échéant, le procédé suivant l'invention comprend en outre l'étape consistant à :
(5°) enrober chacune des particules lyophilisées (i.e. les corpuscules séchés par cryodessiccation) à l'intérieur d'une membrane polymère à paroi continue.

Lors de la formation des microparticules, l'extrudat (dénommé "paraison" par l'homme du métier) est fragmenté par voie mécanique à la sortie de la tête d'extrusion qui peut comprendre une ou plusieurs filières. La fragmentation peut être effectuée au moyen d'un couteau rotatif ou d'une lame pivotant en va-et-vient, afin d'obtenir des bâtonnets sensiblement cylindriques; elle peut être réalisée également au moyen de vibrations, afin d'obtenir des microbilles sensiblement sphériques.

Selon une variante, on obtient des bâtonnets cylindriques ayant, notamment comme dans l'art antérieur, une longueur de 1 à 5 mm et un diamètre de 1 à 1,5 mm, à partir de filières circulaires, d'une part, et des bâtonnets approximativement cylindriques ou oblongs ayant notamment une longueur de 1 à 5 mm et une épaisseur de 1 à 1,5 mm, à partir de filières allongées, d'autre part. La fragmentation ou découpe est dans ce cas effectuée au moyen d'un couteau ou lame obturant, selon la périodicité retenue, lesdites filières. Quand on utilise des filières allongées, il est possible de prévoir un ou plusieurs affaiblissements de matière sur au moins une surface des bâtonnets afin d'obtenir des produits sécables.

Selon une autre variante, les microbilles sont obtenues par des vibrations ou oscillations périodiques de la tête d'extrusion ou de sa ou de ses filières. Ces vibrations ou oscillations périodiques ont une fréquence notamment comprise entre 50 Hz et 10 000 Hz voire même supérieure à 10 000 Hz ; elles permettent de fractionner le flux de l'extrudat sortant de la tête d'extrusion suivant des volumes identiques donnant des microbilles ayant un diamètre calibré final (i.e. après lyophilisation) compris entre 0,05 et 3 mm et de préférence compris entre 0,1 et 1,8 mm.

Comme suivant l'invention, on préfère les microbilles plutôt que les bâtonnets, ce qui suit concerne, sauf indications contraires, essentiellement l'élaboration des microbilles, l'enseignement relatif auxdites microbilles étant directement transposable aux bâtonnets.

A l'étape (1°) par expression "excipient substantiellement hydrophile", on entend le fait que l'excipient hydrophile intervient dans le mélange homogène à extruder selon une quantité supérieure ou égale à 5 % en poids par rapport au poids dudit mélange, l'ensemble excipient hydrophile/eau représentant une quantité supérieure ou égale à 15 % en poids par rapport audit mélange. En d'autres termes, l'excipient formant la matrice des microbilles est soit totalement constitué d'un excipient hydrophile (cas général), soit comprend une association d'un excipient lipophile et d'un excipient hydrophile (cas particulier d'une matrice élaborée à partir d'une suspension du type huile-dans-eau).

L'excipient hydrophile (b) comprend deux composants essentiels :
(b1) un composant polymère ayant un poids moléculaire supérieur ou égal à 10 000 daltons, gonflant en présence d'eau, et
(b2) un composant hydrosoluble ou hydrodispersable servant de diluant.

Le composant (b1) intervient en tant qu'agent liant au niveau de la formation des microparticules ou microbilles, d'une part, et en tant qu'agent facilitant la désintégration desdites microparticules ou microbilles après lyophilisation au moment de leur utilisation au contact de l'eau ou d'un milieu aqueux, d'autre part.

De façon pratique, ledit composant (b1) sera une substance ayant un poids moléculaire élevé notamment supérieur à 10 000 daltons et appartenant à l'ensemble des colloïdes et polymères gonflables en présence d'eau, d'une part, et leurs mélanges, d'autre part. De façon avantageuse, ledit composant (b1) sera une substance choisie parmi l'ensemble constitué par la gomme arabique, la gomme xanthane, la gomme adragante, les alginates, les pectinates, la polyvinylpyrrolidone, les polyéthylèneglycols, la cellulose, la carboxyméthylcellulose, les éthers de cellulose, la carboxyméthylchitine, le dextrane, le chitosane (qui est obtenu par désacétylation totale ou partielle de la chitine), la gélatine, les polymères et copolymères acryliques et méthacryliques, la silice colloïdale et leurs mélanges.

De préférence, on fera appel à un composant (b1) choisi parmi l'ensemble constitué par la gomme arabique, la gomme xanthane, la polyvinylpyrrolidone, la carboxyméthylcellulose, les éthers de cellulose (notamment la méthyl- éthyl-, propyl-, hydroxyéthyl- ou hydroxypropylcellulose), le dextrane et leurs mélanges.

De façon avantageuse, on préconise d'utiliser 10 à 350 parties en poids de composant (b1) pour 100 parties en poids d'ingrédient actif.

Le composant (b2) intervient en tant que diluant (ou ballast) physiologiquement inerte de l'ingrédient actif et sert pour la cohésion des microparticules ou microbilles lors de la formation et de leur stockage avant utilisation. En d'autres termes, il contribue à donner du "corps" auxdites microparticules ou microbilles, dès lors que la teneur en ingrédient actif présente dans le matériau lyophilisé final petit être faible. Eu égard à son caractère hydrosoluble ou hydrodispersable, le composant (b2) a une action favorable sur la désintégration desdites microparticules ou microbilles lyophilisées. De façon avantageuse, ledit composant (b2) sera une substance choisie parmi l'ensemble constitué par les sucres, les dextrines et leurs mélanges.

De préférence, on fera appel à un composant (b2) choisi parmi l'ensemble constitué par le lactose, le glycocolle le mannitol, le glucose, le saccharose, la maltodextrine, la cyclodextrine et ses dérivés, les édulcorants de synthèse (notamment l'aspartam et les autres dipeptides analogues, les cyclamates et les saccharinates), les aromes naturels ou synthétiques, et leurs mélanges.

Par "cyclodextrine" on entend ici tout composé du type cycloamylose [voir Merck Index, (1989), 11è édition, page 425, entrée "Cyclodextrins" (No 2724)], en particulier l'α-cyclodextrine ou cyclohexaamylose de formule brute C₃₆H₆₀O₃₀, la β-cyclodextrine ou cycloheptaamylose de formule brute C₄₂H₇₀O₃₅, et la γ-cyclodextrine ou cyclooctaamylose de formule brute C₄₈H₈₀0₄₀.

Par "dérivés de cyclodextrine", on entend ici tout composé cyclodextrine dont un au moins des groupes OH est éthérifié ou estérifié. Lesdits dérivés de cyclodextrine englobent notamment les éthers dans lesquels l'atome d'hydrogène d'au moins un groupe OH, est remplacé par un groupe alkyle en C₁-C₄ ou hydroxyalkyle en C₁-C₄, à savoir en particulier les hydroxyéthyl-cyclodextrines, hydroxypropyl-cyclodextrines et diméthylcyclodextrines.

De façon avantageuse, on préconise d'utiliser 5 à 350 parties en poids de composant (b2) pour 100 parties en poids d'ingrédient actif.

Le cas échéant, l'excipient hydrophile suivant l'invention peut comporter un troisième composant (b3) qui est un agent tensio-actif de caractère hydrophile. Parmi les composés tensio-actifs qui conviennent, on peut citer notamment les tensio-actifs classiques en galénique et pouvant être administrés par voie orale, et en particulier les polysorbates, les esters de sorbitan, les polyéthers de glycérides gras, les lécithines, le laurylsulfate de sodium, le dioctylsulfosuccinate de sodium et leurs mélanges.

La quantité de composant tensio-actif (b3) à utiliser à l'étape (1°) n'est pas critique. Quand ledit composant (b3) est introduit dans le mélange pâteux à extruder, c'est le cas principalement d'un mélange contenant un ingrédient actif non hydrosoluble ou non hydrodispersable, il interviendra généralement suivant une quantité de 0,05 à 3 parties en poids pour 100 parties en poids d'ingrédient actif.

L'ingrédient actif qui intervient à l'étape (1°) peut être liquide ou pulvérulent, il peut être également soit soluble dans l'eau soit insoluble dans l'eau. Quand il est pulvérulent, sa granulométrie sera comprise entre 1 et 1000 µm. Comme une granulométrie trop importante (par exemple supérieure ou égale à 500 µm) ne permet pas d'obtenir les tailles les plus faibles des microparticules ou microbilles lyophilisées suivant l'invention, quand ledit ingrédient actif est insoluble dans l'eau, on recommande de faire appel à des poudres d'ingrédient actif ayant une granulométrie comprise entre 1 et 200 µm. On obtient des poudres d'une granulométrie de 1-30 µm par micronisation à jet d'air et d'une granulométrie de 30-200 µm par broyage. Quand l'ingrédient actif pulvérulent n'est pas soluble dans l'eau, il sera incorporé dans le mélange devant être extrudé de façon à obtenir une émulsion notamment du type huile-dans-eau, ledit ingrédient actif étant placé alors soit dans la phase aqueuse soit dans la phase huileuse lors de la préparation dudit mélange.

L'eau est ajoutée à l'étape (1°) pour permettre la lyophilisation ultérieure, d'une part, et surtout, régler la viscosité du mélange pâteux, d'autre part. Selon une caractéristique de l'invention, il est essentiel que le mélange pâteux soumis à l'extrusion ait une viscosité inférieure à 1 Pa.s. De façon pratique, on recommande que ledit mélange que l'on va extruder ait une viscosité comprise entre 0,1 et 0,3 Pa.s à la température ambiante, c'est-à-dire une viscosité différente de celle préconisée dans DE-B-2 725 924 (à savoir 0,01-0,02 Pa.s comme rappelé plus haut).

Il est important que ledit mélange pâteux contenant de l'eau, que l'on va extruder, soit homogène ; si nécessaire, après son obtention un tel mélange insuffisamment homogène pourra être "lissé" par passage au travers d'un dispositif homogénéisateur.

L'extrusion de l'étape (2°) est réalisée à la température ambiante comme indiqué ci-dessus. En d'autres termes, pour avoir une telle température, la température de la tête d'extrusion sera réglée à 15-20°C. Cette extrusion consiste à faire forcer le passage du mélange pâteux à travers une tête d'extrusion comportant une ou plusieurs filières de diamètre donné, ladite tête d'extrusion étant soumise à des vibrations ou oscillations périodiques pour former des gouttelettes sphériques.

Suivant une autre caractéristique de l'invention, il est important de congeler relativement rapidement les gouttelettes pour les "fixer" dans la forme qui est la leur après fragmentation ou découpe (ici par vibrations) à la sortie de la tête d'extrusion. Ainsi, la congélation de l'étape (3°) est réalisée au moyen d'un courant gazeux inerte (notamment de l'azote ou de l'argon) ayant une température inférieure à 0°C et de préférence inférieure à -10°C. Les gouttelettes tombent par gravité (ou par surpression, ce qui revient au même) tandis que le courant gazeux congelant va (de préférence) à contre-courant du flux (ou trajet) desdites gouttelettes.

La congélation de l'étape (3°) au moyen d'un courant gazeux inerte constitue soit le début de la phase de congélation de la lyophilisation soit la totalité de ladite phase.

La phase congélation de la lyophilisation comprend le refroidissement de la masse à lyophiliser à une température comprise entre -18 et -80°C et de préférence à une température de -30 à -50°C. Aussi, si par exemple le courant gazeux inerte initie la congélation en refroidissant les gouttelettes à -12°C, on poursuit et complète la congélation au niveau du lyophilisateur jusqu'à une température inférieure ou égale à -18°C (voire inférieure ou égale à -30°C, comme indiqué ci-dessus). En revanche, si le courant gazeux inerte refroidit lesdites gouttelettes à une température de -45°C par exemple, il n'y a pas lieu de poursuivre la congélation dans le lyophilisateur.

A l'étape (4°), la phase de sublimation de l'eau dans la technique de lyophilisation met en oeuvre de façon classique, un gradient de températures et un gradient de pressions réduites, (i) pour aller de la température de congélation sous 1 bar à la température de 25-40°C sous 0,3 mbar (en 1 à 2 heures), puis (ii) pour poursuivre la sublimation pendant 10 minutes à 25-40°C sous 0,05 mbar.

De façon avantageuse, la lyophilisation est réalisée en utilisant des plateaux garnis d'une monocouche de gouttelettes congelées.

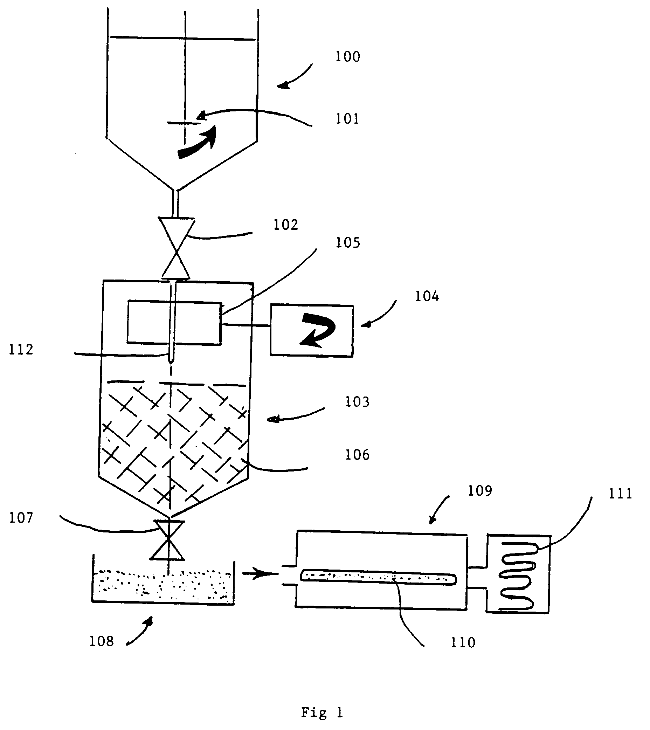

L'installation de la figure 1 pour mettre en oeuvre le procédé de l'invention comprend schématiquement un mélangeur 100 pourvu d'un agitateur rotatif 101 dans lequel est préparé le mélange du composant (a), l'ingrédient actif, des composants de l'excipient, y compris les composants (b1), (b2) et le cas échéant (b3), et du composant (c), l'eau. Dans ce mélange, après homogénéisation, l'ingrédient actif est sous forme de solution, suspension ou émulsion.

Par l'intermédiaire d'un dispositif 102, le mélangeur 100 alimente une enceinte 103 dans laquelle est disposé un dispositif de vibrations à oscillations périodiques 105 lié à la tête d'extrusion. Ces vibrations sont produites par un générateur de fréquence 104.

D'une filière 112, des gouttelettes (non représentées) tombent par gravité vers le fond de l'enceinte 103 pour former l'ensemble 106. Sous l'action d'un courant de gaz inerte (non représenté) froid, ascendant (i.e. circulant à contre-courant vis-à-vis du flux des gouttelettes), lesdites gouttelettes sont congelées (par exemple à -15°C). Par l'intermédiaire d'un dispositif 107, les gouttelettes congelées sont chargées sur un plateau de lyophilisation 108 puis introduites dans un lyophilisateur 109 comprenant notamment un tunnel 110 et un dispositif 111 de refroidissement (fin de la phase dite de congélation de -18 à -80°C) et de chauffage (phase de sublimation).

De plus des orifices (non représentés) pour l'évacuation du courant gazeux de refroidissement sont prévus au sommet de l'enceinte 103 et/ou latéralement au niveau ou légèrement en-dessous de la tête d'extrusion.

Si nécessaire, la portion de l'enceinte 103 contenant l'ensemble des gouttelettes congelées 106, le dispositif 107 et le plateau 108 peuvent être logés à l'intérieur d'une enceinte réfrigérante (non représentée).

Le cas échéant, pour éviter, si cela est nécessaire, la désintégration des gouttelettes, d'une part, et leur agglomération (entre elles ou sur les parois), d'autre part, le contenu de l'ensemble des éléments 106, 107 et 108 ou d'au moins un d'entre eux (notamment 106 et 108) peut être placé dans un lit fluidisé. Un tel lit fluidisé est avantageux pour obtenir en particulier un remplissage du plateau 108 sous la forme d'une monocouche de gouttelettes congelées.

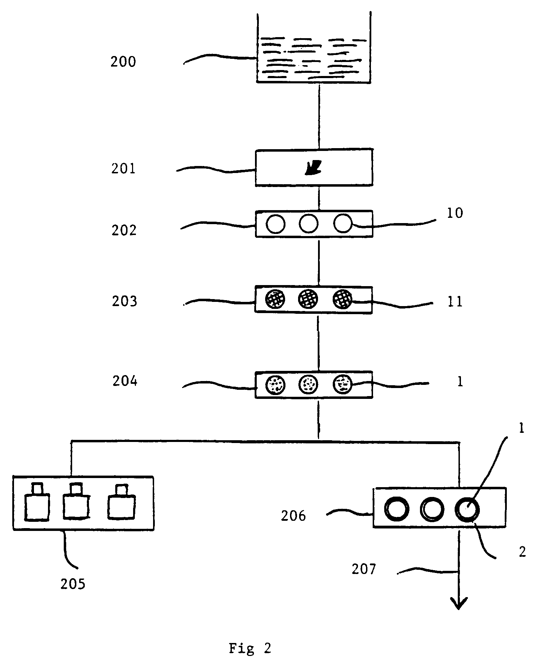

La figure 2 est un diagramme illustrant un mode de mise en oeuvre du procédé de l'invention pour la préparation de microbilles. Ce diagramme comprend :
- en 200, la préparation du mélange contenant l'ingrédient actif (a) les composants hydrophile (b1), (b2) et le cas échéant (b3) de l'excipient physiologiquement acceptable, et de l'eau, ce mélange ayant une viscosité comprise entre 0,1 et 0,3 Pa.s ;
- en 201, l'extrusion de ce mélange sous vibrations pour obtenir des gouttelettes ;
- en 202, les gouttelettes isolées 10 tombant par gravité ;
- en 203, la congélation des gouttelettes 10 au moyen du contre-courant gazeux inerte, afin d'obtenir des gouttelettes isolées et congelées 11 ;
- en 204, la lyophilisation des gouttelettes isolées et congelées 11 pour obtenir des microbilles lyophilisées 1, cette lyophilisation comprend ici la poursuite de la congélation puis la sublimation de l'eau ;
- les microbilles lyophilisées 1 obtenues en 204 sont ensuite soit (i) conditionnées en 205 notamment dans des flacons, soit (ii) enrobées en 206 par une enveloppe polymère 2 à paroi continue et de préférence poreuse puis dirigées selon la flèche 207 vers le conditionnement notamment dans des flacons comme en 205.

A l'étape (5°) on procède, si cela est plutôt préférable, à l'enrobage des microbilles isolées, obtenues selon le procédé de l'invention par extrusion puis lyophilisation. Comme indiqué plus haut, l'enrobage des lyophilisats à la manière des comprimés n'est pas réalisable, même si l'on sait incorporer dans les lyophilisats oraux des produits enrobés. En revanche, il est tout à fait envisageable d'enrober les microbilles notamment dans le but de masquer le goût des produits amers ou désagréables, de prolonger ou modifier la libération des principes actifs ou de protéger des ingrédients actifs sensibles des agents de dégradation externes.

Les microbilles lyophilisées peuvent être enrobées par divers agents gastrosolubles soit en milieu organique, soit en milieu aqueux avec une isolation préalable. L'enrobage peut se faire par pulvérisation dans un appareil à lit d'air fluidisé, notamment du type GLATT ou WÜRSTER.

Parmi les agents d'enrobage, on peut citer notamment :
- les dérivés cellulosiques, semi-synthétiques tels que en particulier la méthylcellulose, l'éthylcellulose, l'hydroxypropylméthylcellulose, le phtalate d'hydroxypropylcellulose et le phtalate/acétate de cellulose,
- les esters de l'acide polyacrylique et de l'acide polyméthacrylique,
- les copolymères d'éthylène/acétate de vinyle,
- les polyméthylsiloxannes,
- les polyacrylamides,
- la polyvinylpyrrolidone et le poly(acétate de vinyle),
- les acides polylactiques, polyglycoliques et leurs copolymères,
- le polyuréthane,
- les polypeptides,
- etc.

L'enrobage d'une microbille lyophilisée au moyen d'une membrane semiperméable microporeuse obtenue par incorporation de produits solubles dans le film polymérique à paroi continue conduit à la formation d'une sorte de pompe osmotique élémentaire sans apport supplémentaire d'agent osmotique, tel que notamment les sels de sodium et de potassium ; le produit lyophilisé étant par définition très hydrophile a tendance, quand il est placé en présence d'eau, à faire appel à un apport d'eau de l'extérieur : l'eau dissout l'ingrédient actif et il se crée une pression osmotique qui expulse la solution aqueuse contenant l'ingrédient actif.

Le produit solubilisé par l'eau va sortir de façon progressive au fur et à mesure que l'eau pénètre dans la microbille. On obtient ainsi une libération régulière de la substance active. Par une formulation appropriée du lyophilisat oral et un choix judicieux de la membrane on peut moduler "à la demande" la libération des ingrédients actifs ce qui permet notamment une chronothérapie efficace.

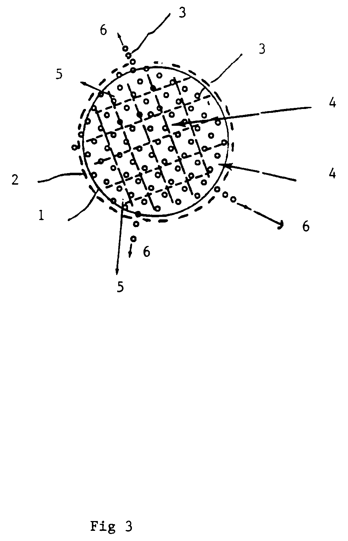

La figure 3 illustre schématiquement le fonctionnement des microbilles lyophilisées et enrobées en tant que "pompes" ou "réservoirs" osmotiques. La microbille 1, qui comprend ici un ingrédient actif sous forme de microparticules 3, est enrobée par une pellicule ou enveloppe 2 semi-perméable. Après administration orale, l'eau ou le liquide aqueux corporel (dans le cas d'espèce, le suc gastrique) pénètre l'enveloppe 2 selon les flèches 4, puis en ressort suivant les flèches 5 en entraînant l'ingrédient actif 3 suivant les flèches 6. Ce mode de fonctionnement convient dans le cas où l'ingrédient actif est soluble dans l'eau et les liquides corporels contenant de l'eau.

En variante, on peut prévoir un enrobage gastro-résistant, pour libération de l'ingrédient actif au niveau entérique.

### MEILLEUR MODE

Le meilleur mode de mise en oeuvre du procédé suivant l'invention consiste à préparer des microbilles ayant un diamètre calibré dans la gamme 0,1-1,8 mm en procédant comme suit :
(1°) la préparation (à la température ambiante) d'un mélange homogène ayant une viscosité comprise entre 0,1 et 0,3 Pa.s à partir
   (a) de 100 parties en poids d'un ingrédient actif,
   (b) d'un excipient substantiellement hydrophile contenant
      (b1) 10 à 350 parties en poids d'un composant polymère ayant un poids moléculaire supérieur ou égal à 10 000 daltons, gonflant dans l'eau et choisi parmi l'ensemble constitué par la gomme arabique, la gomme xanthane, la polyvinylpyrrolidone, la carboxyméthylcellulose, les éthers de cellulose (notamment la méthyl-, éthyl-, propyl-, hydroxyéthyl- ou hydroxypropylcellulose), le dextrane, et leurs mélanges,
      (b2) 5 à 350 parties en poids d'un composant hydrosoluble ou hydrodispersable servant de diluant et choisi parmi l'ensemble constitué par le lactose, le glycocolle, le mannitol, le glucose, le saccharose, la maltodextrine, la cyclodextrine et ses dérivés, les édulcorants de synthèse (notamment l'aspartam et les autres dipeptides analogues, les cyclamates et les saccharinates), les arômes naturels ou synthétiques, et leurs mélanges, et
      (b3) le cas échéant, 0,05 à 3 parties en poids d'un composant tensio-actif, et
   (c) de l'eau en quantité suffisante pour obtenir ladite viscosité de 0,1-0,3 Pa.s ;
(2°) l'extrusion du mélange, ainsi obtenu, à la température ambiante (15-20°C) et la fragmentation de l'extrudat au moyen de vibrations d'une fréquence de 50 à 10 000 Hz (ces vibrations étant notamment appliquées à la tête d'extrusion) ;
(3°) la congélation des gouttelettes, ainsi obtenues, au moyen d'un contre-courant gazeux inerte tandis que lesdites gouttelettes tombent par gravité, la congélation étant poursuivie dans un lyophilisateur jusqu'à une température de -30°C à -50°C ; et,
(4°) l'élimination de l'eau dans ledit lyophilisateur par sublimation.

Suivant ce meilleur mode, le recueil des gouttelettes congelées par le contre-courant est fait sous la forme d' une monocouche, la lyophilisation étant également réalisée sous la forme d'un chargement de gouttelettes constituant également une monocouche.

En ce qui concerne l'utilisation des microparticules lyophilisées de l'invention, il convient de remarquer que
- en thérapeutique humaine et vétérinaire, notamment chez les animaux à sang chaud tels que les mammifères, on préconise des microbilles éventuellement enrobées (ayant un diamètre calibré compris entre 0,1 et 1,8 mm) destinées à être administrées par voie orale ;
- en cosmétique, on préconise des microbilles enrobées (ayant un diamètre calibré compris entre 0,05 et 0,5 mm) destinées à être incorporées dans des préparations du type crème, onguent ou lotion, l'enrobage desdites microbilles pouvant contenir du collagène (ou un collagène modifié) clivable par les collagénases contenues dans la transpiration ;
- en diététique humaine, on préconise des microbilles ou des bâtonnets ; et
- dans le domaine alimentaire tant chez l'homme que l'animal, on préconise des bâtonnets contenant en tant qu'ingrédient actif un antibiotique, un facteur de croissance, un aminoacide, un peptide ou un de leurs mélanges.

D'autres avantages et caractéristiques de l'invention seront mieux compris à la lecture qui va suivre d'exemples de réalisation nullement limitatifs mais donnés à titre d'illustration.

### EXEMPLE 1 Microbilles de paracétamol

On prépare un mélange à extruder ayant la formulation suivante :

| | |
|---|---|
| Paracétamol | 100,00 g |
| Dextran 70 000 | 10,00 g |
| Gomme xanthane | 0,05 g |
| Lactose | 15,00 g |
| Polysorbate 60 | 0,40 g |
| Eau | 120,00 g |

On dissout dans l'eau le lactose, le polysorbate 60 et le dextran 70 000, ajoute le paracétamol (de granulométrie 50-200 µm), disperse au moyen d'un homogénéisateur opérant à une vitesse angulaire de 2 000 tours/minute, pendant 2 minutes. On obtient un mélange ayant une viscosité comprise entre 0,1 et 0,3 Pa.s à la température ambiante.

On introduit ce mélange dans une extrudeuse dont la tête d'extrusion comporte plusieurs filières ayant chacune un orifice de 0,5 mm de diamètre et soumise chacune à des vibrations d'une fréquence de 100 Hz. Les gouttelettes formées sont congelées au moyen d'un contre-courant d'azote puis portées (au niveau de la phase congélation d'un lyophilisateur), sous forme d'une monocouche, à une température comprise entre -40°C et -45°C.

L'eau des gouttelettes congelées est éliminée par sublimation, par réchauffement jusqu'à +35°C et une pression de 0,300 mbar, puis pendant 10 minutes à +35°C jusqu'à une pression de 0,050 mbar.

Les microbilles lyophilisées ainsi obtenues ont une excellente tenue mécanique et présentent une granulométrie de 1,200 mm.

### EXEMPLE 2 Microbilles de probucol

A partir d'un mélange ayant la formulation suivante:

| | |
|---|---|
| Probucol | 100 g |
| Laurylsulfate de sodium | 2 g |
| Dextran 70 | 13 g |
| Lactose | 30 g |
| Eau | 300 g |

on prépare des microbilles selon les modalités décrites à l'exemple 1, avec les différences que la granulométrie du probucol est de 2 à 10 µm et les filières ont un diamètre de 0,6 mm. On obtient des microbilles lyophilisées ayant un diamètre de 1,5 mm.

### EXEMPLE 3 Microbilles de piroxicam

A partir d'un mélange ayant la formulation suivante:

| | |
|---|---|
| Piroxicam | 2,00 g |
| Dextran 70 | 6,00 g |
| Gomme xanthane | 0,05 g |
| Lactose | 6,00 g |
| Tween 60 | 0,10 g |
| Eau | 500,00 g |

on prépare des microbilles selon les modalités opératoires décrites à l'exemple 1 avec les différences que la granulométrie du piroxicam est de 2 à 5 µm et que les filières ont chacune un diamètre de 0,2 mm. On obtient des microbilles lyophilisées ayant un diamètre de 0,500 mm.

### EXEMPLE 4 Microbilles de phloroglucinol

A partir d'un mélange ayant la formulation suivante:

| | |
|---|---|
| Phloroglucinol | 80 g |
| Dextran 70 | 20 g |
| Aspartam | 2 g |
| Mannitol | 10 g |
| Eau | 500 g |

on prépare selon les modalités de l'exemple 3 des microbilles lyophilisées ayant un diamètre de 0,500 mm.

### EXEMPLE 5 Microbilles de tiadénol

A partir d'un mélange ayant la formulation suivante:

| Phase A : | |
|---|---|
| Tiadénol | 100 g |
| Polysorbate 60 | 2 g |
| Polyéthoxyéther d'acide gras | 2 g |
| Miglyol 812 | 20 g |

| Phase B : | |
|---|---|
| Dextran 70 | 10 g |
| Lactose | 5 g |
| Eau | 50 g |

on prépare des microbilles selon le protocole qui comprend la préparation du mélange de la phase A à 70°C et celle de la phase B à 70°C, le mélange de A et de B, l'homogénéisation du mélange résultant au moyen d'un appareil du type Turrax opérant à une vitesse angulaire de 5 000 tours/minute pendant 5 minutes, l'introduction du mélange homogène résultant dans l'extrudeuse puis la reproduction des modalités opératoires décrites à l'exemple 2. Les microbilles lyophilisées obtenues ont une granulométrie de 1,5 mm.

### EXEMPLE 6 Enrobage

On a procédé à l'enrobage des microbilles obtenues à l'exemple 1 pour masquer leur arrière goût au moyen de la solution d'enrobage suivante :

| | |
|---|---|
| Ethylcellulose | 12,5 % en poids |
| Lactose | 10 % en poids |
| Phtalate de dibutyle | 1 % en poids |
| Isopropanol | 66,5 % en poids |

Dans un appareil à lit d'air fluidisé du type GLATT WSG 5 on introduit 1 kg de microbilles on chauffe l'appareil à 50-55°C de façon à obtenir une température dans le produit enrobé de 30-35°C. On pulvérise la solution d'enrobage décrite ci-dessus suivant un débit de 2-4 mg/mn et une pression de nébulisation de 2 bars, pendant 120 minutes avec séchage final de 30 minutes. On obtient un enrobage représentant environ 5 % en poids du poids de chaque microbille. Le produit ainsi enrobé a un goût neutre et il convient pour une administration par voie orale sans modification sensible de sa vitesse de libération au niveau de l'estomac.

### EXEMPLE 7 Solution d'enrobage

Avec une solution d'enrobage ayant pour formulation:

| | |
|---|---|
| Eudragit L 100 | 7,3 % en poids |
| Phtalate de dibutyle | 1,5 % en poids |
| Talc | 1,8 % en poids |
| Isopropanol | 89,4 % en poids |

on obtient un enrobage entérique pour les microbilles de l'exemple 2, l'enrobage représentant 5 à 10 % en poids par rapport au poids desdites microbilles.

### EXEMPLES 8-12

En procédant selon les modalités opératoires décrites à l'exemple 1 et en ne faisant varier que la fréquence des vibrations, on obtient des microbilles de paracétamol ayant un diamètre de 1,5 mm, 1,0 mm, 0,7 mm, 0,5 mm et respectivement 0,1 mm avec des vibrations de 50 Hz, 500 Hz, 1 000 Hz, 2 000 Hz et respectivement 10 000 Hz.

### EXEMPLES 13-14 Microbilles de flérobutérol

A partir des formulations suivantes

| | Ex 13 | Ex 14 |
|---|---|---|
| Flérobutérol | 3 g | 3 g |
| Lactose | 25 g | - |
| Bétacyclodextrine | - | 30 g |
| Dextran 70 | 25 g | 25 g |
| Saccharinate de sodium | 3 g | 3 g |
| Eau | 100 g | 100 g |

on prépare des microbilles selon l'invention.

| | |
|---|---|
| Diamètre des filières | : 0,2 mm |
| Diamètre des microbilles | : 0,5 mm |

### EXEMPLE 15 Microbilles de carbinoxamine

A partir de la formulation suivante

| | |
|---|---|
| Maléate de carbinoxamine | 20 g |
| Glycocolle | 20 g |
| Dextran 70 | 10 g |
| Aspartam | 5 g |
| Gomme xanthane | 1 g |
| Eau | 100 g |

on prépare des microbilles selon l'invention.

| | |
|---|---|
| Diamètre des filières | : 0,2 mm |
| Diamètre des microbilles | : 0,5 mm |

### EXEMPLES 16-17 Microbilles de modafinil

A partir des formulations suivantes

| | Ex 16 | Ex 17 |
|---|---|---|
| Modafinil* | 100 g | 100 g |
| Saccharinate de sodium | 2 g | 2 g |
| Dextran 70 | 10 g | 10 g |
| Tween 80 | 2 g | 2 g |
| Hydroxypropyl-β-cyclodextrine | 100 g | - |
| Lactose ou mannitol | - | 40 g |
| Gomme xanthane | 1 g | 1 g |
| Eau | 200 g | 200 g |

| | | |
|---|---|---|
| Note (*) granulométrie du modafinil : 2-5 µm , | | |

on prépare des microbilles selon l'invention.

| | |
|---|---|
| Diamètre des filières | : 0,5mm |
| Diamètre des microbilles | : 1 mm |

### EXEMPLES 18-19 Microbilles de dexfenfluramine

A partir des formulations suivantes

| | Ex 18 | Ex 19 |
|---|---|---|
| Dexfenfluramine* | 300 g | 300 g |
| Dextran 70 | 20 g | 20 g |
| Gomme xanthane | 0,5 g | 0,5 g |
| Acide citrique | 5 g | 5 g |
| Aspartam | 6 g | 6 g |
| Mannitol | 50 g | - |
| Bétacyclodextrine | - | 50 g |
| Eau | 300 g | 300 g |

| | | |
|---|---|---|
| Note (*) granulométrie de la dexfenfluramine : 5-10 µm , | | |

on prépare des microbilles selon l'invention.

| | |
|---|---|
| Diamètre des filières | : 0,5 mm |
| Diamètre des microbilles | : 1,2 mm |

### EXEMPLE 20 Microbilles de lopéramide

A partir de la formulation suivante

| | |
|---|---|
| Lopéramide | 2 g |
| Aspartam | 20 g |
| Dextran 70 | 20 g |
| Tween 60 | 1 g |
| Gomme xanthane | 1 g |
| Mannitol | 20 g |
| Eau | 100 g |

on prépare des microbilles selon l'invention.

| | |
|---|---|
| Diamètre des filières | : 0,5 mm |
| Diamètre des microbilles | : 1 mm |

### EXEMPLE 21 Microbilles de lorazépam

A partir de la formulation suivante

| | |
|---|---|
| Lorazépam | 2,5 g |
| Aspartam | 8 g |
| Polyvinylpyrrolidone | 25 g |
| Tween 60 | 1 g |
| Gomme xanthane | 1 g |
| Diméthyl-β-cyclodextrine | 40 g |
| Eau | 100 g |

on prépare des microbilles selon l'invention.

| | |
|---|---|
| Diamètre des filières | : 0,2 mm |
| Diamètre des microbilles | : 0,5 mm. |

## Revendications

1. Procédé pour la préparation de particules comprenant chacune un excipient formant une matrice et au moins un ingrédient actif régulièrement réparti dans la masse de ladite matrice, ledit procédé, qui comprend les opérations d'extrusion puis de lyophilisation, étant caractérisé en ce qu'il comprend les étapes consistant dans :
(1°) la préparation d'un mélange homogène à partir
(a) d'au moins un ingrédient actif,
(b) d'un excipient hydrophile physiologiquement acceptable, et
(c) d'eau,
de façon à obtenir un mélange pâteux ayant une viscosité, mesurée à la température ambiante (15-20°C), inférieure à 1 Pa.s ;
(2°) l'extrusion du mélange homogène, ainsi obtenu, et la découpe de l'extrudat pour obtenir des corpuscules humides ;
(3°) la congélation desdits corpuscules, ainsi obtenus, pendant qu'ils tombent par gravité dans un courant gazeux inerte ayant une température inférieure à 0°C ; et,
(4°) le séchage desdits corpuscules par cryodessiccation.

2. Procédé suivant la revendication 1, caractérisé en ce que l'extrusion est réalisée à la température ambiante (15-20°C).

3. Procédé suivant la revendication 1, caractérisé en ce que le mélange pâteux contenant de l'eau a une viscosité comprise entre 0,1 et 0,3 Pa.s à 15-20°C.

4. Procédé suivant la revendication 1, caractérisé en ce que l'excipient hydrophile (b) comprend deux composants essentiels :
(b1) un composant polymère ayant un poids moléculaire supérieur ou égal à 10 000 daltons, gonflant en présence d'eau, et
(b2) un composant hydrosoluble ou hydrodispersable servant de diluant.

5. Procédé suivant la revendication 4, caractérisé en ce que le composant (b1) est une substance choisie parmi l'ensemble constitué par la gomme arabique, la gomme xanthane, la gomme adragante, les alginates, les pectinates, la polyvinylpyrrolidone, les polyéthylèneglycols, la cellulose, la carboxyméthylcellulose, les éthers de cellulose, la carboxyméthylchitine, le dextrane, le chitosane, la gélatine, les polymères et copolymères acryliques et méthacryliques, la silice colloïdale et leurs mélanges.

6. Procédé suivant la revendication 4, caractérisé en ce que le composant (b2) est une substance choisie parmi l'ensemble constitué par les sucres et les dextrines.

7. Procédé suivant la revendication 4, caractérisé en ce que le composant (b2) est choisi parmi l'ensemble constitué par le lactose, le glycocolle, le mannitol, le glucose, le saccharose, la maltodextrine, la cyclodextrine et ses dérivés, les édulcorants de synthèse, les aromes naturels ou synthétiques, et leurs mélanges.

8. Procédé suivant la revendication 1, caractérisé en ce qu'à l'étape (1°) on utilise
(a) 100 parties en poids d'un ingrédient actif,
(b1) 10 à 350 parties en poids de substance polymère gonflant dans l'eau, et
(b2) 5 à 350 parties en poids d'une substance choisie parmi les sucres, les dextrines, et leurs mélanges.

9. Procédé suivant la revendication 1, caractérisé en ce qu'à l'étape (1°) on utilise en outre
(b3) un composant tensio-actif, notamment à raison de 0,05 à 3 parties en poids dudit composant tensio-actif pour 100 parties en poids d'ingrédient actif.

10. Procédé suivant la revendication 1, caractérisé en ce que la congélation de l'étape (3°) est initiée par ledit courant gazeux inerte circulant à contre-courant du trajet des corpuscules humides puis poursuivie jusqu'à une température située dans la gamme de -18 à -80°C (de préférence de -30 à -50°C) dans un lyophilisateur.

11. Procédé suivant la revendication 1, caractérisé en ce qu'il comprend en outre, après l'étape (4°), l'étape consistant à : (5°) enrober chacune des particules lyophilisées, ainsi obtenues, à l'intérieur d'une membrane polymère à paroi continue.

12. Procédé suivant la revendication 1, caractérisé en ce qu'il comprend les étapes consistant dans
(1°) la préparation à la température ambiante d'un mélange homogène ayant une viscosité comprise entre 0,1 et 0,3 Pa.s, à partir
(a) de 100 parties en poids d'un ingrédient actif,
(b) d'un excipient substantiellement hydrophile contenant
(b1) 10 à 350 parties en poids d'un composant polymère ayant un poids moléculaire supérieur ou égal à 10 000 daltons, gonflant dans l'eau et choisi parmi l'ensemble constitué par la gomme arabique, la gomme xanthane, la polyvinylpyrrolidone, la carboxyméthylcellulose, les éthers de cellulose, le dextrane et leurs mélanges,
(b2) 5 à 350 parties en poids d'un composant hydrosoluble ou hydrodispersable servant de diluant et choisi parmi l'ensemble constitué par le lactose, le glycocolle, le mannitol, le glucose, le saccharose, la maltodextrine, la cyclodextrine et ses dérivés, les aromes naturels ou synthétiques, et leurs mélanges, et
(b3) le cas échéant, 0,05 à 3 parties en poids d'un composant tensioactif, et
(c) de l'eau en quantité suffisante pour obtenir ladite viscosité de 0,1-0,3 Pa.s ;
(2°) l'extrusion du mélange, ainsi obtenu, à la température ambiance (15-20°C) et la fragmentation de l'extrudat sous forme de gouttelettes au moyen de vibrations d'une fréquence de 50 à 10 000 Hz ;
(3°) la congélation des gouttelettes, ainsi obtenues, au moyen d'un contre-courant gazeux inerte tandis que lesdites gouttelettes tombent par gravité, la congélation étant poursuivie dans un lyophilisateur jusqu'à une température de -30°C à -50°C ; et,
(4°) l'élimination de l'eau dans ledit lyophilisateur par sublimation.

13. Procédé suivant la revendication 1, caractérisé en ce que, à l'étape (1°) l'ingrédient actif est choisi parmi l'ensemble constitué par le paracétamol, le probucol, le piroxicam, le phloroglucinol, le tiadénol, le flérobutérol, le modafinil, la dexfenfluramine, le maléate de carbinoxamine, le lopéramide, le lorazépam, et leurs mélanges.

## Claims

1. A process for the preparation of particles each comprising an excipient forming a matrix and at least one active ingredient uniformly distributed in the mass of said matrix, said process, which comprises the operations of extrusion and then lyophilization, being characterized in that it comprises the steps consisting of
(1°) the preparation of a homogeneous mixture from
(a) at least one active ingredient,
(b) a physiologically acceptable hydrophilic excipient, and
(c) water
to give a pasty mixture with a viscosity below 1 Pa.s, measured at room temperature (15-20°C);
(2°) the extrusion of the resulting homogeneous mixture and the cutting of the extrudate to give moist particles;
(3°) the freezing of the resulting particles as they fall under gravity through a stream of inert gas at a temperature below 0°C; and
(4°) the drying of said particles by freeze drying.

2. A process according to claim 1 characterized in that the extrusion is carried out at room temperature (15-20°C).

3. A process according to claim 1 characterized in that the pasty mixture containing water has a viscosity of between 0.1 and 0.3 Pa.s at 15-20°C.

4. A process according to claim 1 characterized in that the hydrophilic excipient (b) comprises two essential components:
(b1) a polymer component with a molecular weight greater than or equal to 10,000 daltons, which swells in the presence of water, and
(b2) a water-soluble or water-dispersible component which acts as a diluent.

5. A process according to claim 4 characterized in that component (b1) is a substance selected from the group consisting of gum arabic, xanthan gum, gum tragacanth, alginates, pectinates, polyvinylpyrrolidone, polyethylene glycols, cellulose, carboxymethyl cellulose, cellulose ethers, carboxymethyl chitin, dextran, chitosan, gelatin, acrylic and methacrylic polymers and copolymers, colloidal silica and mixtures thereof.

6. A process according to claim 4 characterized in that component (b2) is a substance selected from the group consisting of sugars and dextrins.

7. A process according to claim 4 characterized in that component (b2) is selected from the group consisting of lactose, glycocoll, mannitol, glucose, sucrose, maltodextrin, cyclodextrin and derivatives thereof, artificial sweeteners, natural or synthetic flavorings and mixtures thereof.

8. A process according to claim 1 characterized in that
(a) 100 parts by weight of an active ingredient,
(b1) 10 to 350 parts by weight of a polymer substance which swells in water, and
(b2) 5 to 350 parts by weight of a substance selected from sugars, dextrins and mixtures thereof
are used in step (1°).

9. A process according to claim 1 characterized in that
(b3) a surface-active component, especially in an amount of 0.05 to 3 parts by weight of said surface-active component per 100 parts by weight of active ingredient,
is also used in step (1°).

10. A process according to claim 1 characterized in that the freezing of step (3°) is initiated by said stream of inert gas circulating in countercurrent to the path of the moist particles, and then continued down to a temperature in the range -18 to -80°C (preferably -30 to -50°C) in a lyophilizer.

11. A process according to claim 1 characterized in that it also comprises, after step (4°), the step consisting of
(5°) the coating of each of the resulting lyophilized particles with a continuous-wall polymer membrane.

12. A process according to claim 1 characterized in that it comprises the steps consisting of
(1°) the preparation at room temperature of a homogeneous mixture with a viscosity of between 0.1 and 0.3 Pa.s from
(a) 100 parts by weight of an active ingredient,
(b) a substantially hydrophilic excipient containing
(b1) 10 to 350 parts by weight of a polymer component with a molecular weight greater than or equal to 10,000 daltons, which swells in water and is selected from the group consisting of gum arabic, xanthan gum, polyvinylpyrrolidone, carboxymethyl cellulose, cellulose ethers, dextran and mixtures thereof,
(b2) 5 to 350 parts by weight of a water-soluble or water-dispersible component which acts as a diluent and is selected from the group consisting of lactose, glycocoll, mannitol, glucose, sucrose, maltodextrin, cyclodextrin and derivatives thereof, natural or synthetic flavorings and mixtures thereof, and
(b3) if appropriate, 0.05 to 3 parts by weight of a surface-active component, and
(c) a sufficient amount of water to obtain said viscosity of 0.1-0.3 Pa.s;
(2°) the extrusion of the resulting mixture at room temperature (15-20°C) and the fragmentation of the extrudate into droplets by means of vibrations at a frequency of 50 to 10,000 Hz;
(3°) the freezing of the resulting droplets by means of a countercurrent of inert gas as said droplets fall under gravity, the freezing being continued in a lyophilizer down to a temperature of -30°C to -50°C; and
(4°) the removal of the water in said lyophilizer by sublimation.

13. A process according to claim 1 characterized in that, in step (1°), the active ingredient is selected from the group consisting of paracetamol, probucol, piroxicam, phloroglucinol, tiadenol, flerobuterol, modafinil, dexfenfluramine, carbinoxamine maleate, loperamide, lorazepam and mixtures thereof.

## Patentansprüche

1. Verfahren zur Herstellung von Teilchen, die jeweils einen eine Matrix bildenden Träger und mindestens einen gleichmäßig in der Masse dieser Matrix verteilten Wirkstoff enthalten, wobei das Verfahren, das die Schritte einer Extrusion und einer anschließenden Lyophilisation umfaßt, dadurch gekennzeichnet ist, daß es die folgenden Stufen umfaßt:
(1) Herstellung eines homogenen Gemisches aus
(a) mindestens einem Wirkstoff,
(b) einem physiologisch verträglichen hydrophilen Träger und
(c) Wasser
unter Bildung eines pasteusen Gemisches mit einer bei Umgebungstemperatur (15-20°C) gemessenen Viskosität unter 1 Pa.s;
(2) Extrusion des auf diese Weise erhaltenen homogenen Gemisches und Schneiden des Extrudats unter Bildung von feuchten Teilchen;
(3) Einfrieren der auf diese Weise erhaltenen Teilchen, während diese unter Schwerkrafteinwirkung in einen inerten Gasstrom mit einer Temperatur unter 0°C fallen;
(4) Trocknen dieser Teilchen durch kryogene Trocknung.

2. Verfahren nach Anspruch 1, dadurch gekennzeichnet, daß die Extrusion bei Umgebungstemperatur (15-20°C) durchgeführt wird.

3. Verfahren nach Anspruch 1, dadurch gekennzeichnet, daß das pasteuse Gemisch mit einem Gehalt an Wasser eine Viskosität von 0,1 bis 0,3 Pa.s bei 15-20°C aufweist.

4. Verfahren nach Anspruch 1, dadurch gekennzeichnet, daß der hydrophile Träger (b) folgende zwei wesentlichen Bestandteile umfaßt:
(b1) einen polymeren Bestandteil mit einem Molekulargewicht von 10 000 Dalton oder mehr, der in Gegenwart von Wasser quillt, und
(b2) einen als Verdünnungsmittel dienenden, in Wasser löslichen oder in Wasser dispergierbaren Bestandteil.

5. Verfahren nach Anspruch 4, dadurch gekennzeichnet, daß es sich beim Bestandteil (b1) um eine Substanz handelt, die aus der Gruppe Gummi arabicum, Xanthangummi, Tragantgummi, Alginate, Pectinate, Polyvinylpyrrolidon, Polyethylenglycole, Cellulose, Carboxymethylcellulose, Celluloseether, Carboxymethylchitin, Dextran, Chitosan, Gelatine, Acryl- und Methacryl-Polymere und -Copolymere, kolloidales Siliciumdioxid und Gemische davon ausgewählt ist.

6. Verfahren nach Anspruch 4, dadurch gekennzeichnet, daß es sich beim Bestandteil (b2) um eine Substanz handelt, die aus der Gruppe Zucker und Dextrine ausgewählt ist.

7. Verfahren nach Anspruch 4, dadurch gekennzeichnet, daß der Bestandteil (b2) aus der Gruppe Lactose, Glycocoll, Mannit, Glucose, Saccharose, Maltodextrin, Cyclodextrin und dessen Derivate, synthetische Süßstoffe, natürliche oder synthetische Aromastoffe und Gemische davon ausgewählt ist.

8. Verfahren nach Anspruch 1, dadurch gekennzeichnet, daß man in der Stufe (1) folgende Bestandteile verwendet:
(a) 100 Gewichtsteile Wirkstoff,
(b1) 10 bis 350 Gewichtsteile der in Wasser quellenden polymeren Substanz und
(b2) 5 bis 350 Gewichtsteile einer unter Zuckern, Dextrinen und Gemischen davon ausgewählten Substanz.

9. Verfahren nach Anspruch 1, dadurch gekennzeichnet, daß man in der Stufe (1) unter anderem folgenden Bestandteil verwendet:
(b3) einen oberflächenaktiven Bestandteil, insbesondere in einer Menge von 0,05 bis 3 Gewichtsteilen pro 100 Gewichtsteile des Wirkstoffs.

10. Verfahren nach Anspruch 1, dadurch gekennzeichnet, daß das Einfrieren in Stufe (3) durch den inerten Gasstrom eingeleitet wird, der im Gegenstrom zur Bahn der feuchten Teilchen geführt wird, und dann bis zu einer Temperatur im Bereich von -18 bis -80°C (vorzugsweise -30 bis -50°C) in einer Lyophilisationsvorrichtung fortgesetzt wird.

11. Verfahren nach Anspruch 1, dadurch gekennzeichnet, daß es unter anderem nach der Stufe (4) folgende Stufe umfaßt:
(5) Einbringen der einzelnen, auf diese Weise erhaltenen lyophilisierten Teilchen ins Innere einer polymeren Membran mit durchgehender Wand.

12. Verfahren nach Anspruch 1, dadurch gekennzeichnet, daß es folgende Stufen umfaßt:
(1) bei Umgebungstemperatur die Herstellung eines homogenen Gemisches mit einer Viskosität von 0,1 bis 0,3 Pa.s aus
(a) 100 Gewichtsteilen eines Wirkstoffs,
(b) eines im wesentlichen hydrophilen Trägers, enthaltend
(b1) 10 bis 350 Gewichtsteile eines polymeren Bestandteils mit einem Molekulargewicht von 10 000 Dalton oder mehr, der in Wasser quillt und aus der Gruppe Gummi arabicum, Xanthangummi, Polyvinylpyrrolidon, Carboxymethylcellulose, Celluloseether, Dextran und Gemische davon ausgewählt ist,
(b2) 5 bis 350 Gewichtsteile eines in Wasser löslichen oder in Wasser dispergierbaren Bestandteils, der als Verdünnungsmittel dient und aus der Gruppe Lactose, Glycocoll, Mannit, Glucose, Saccharose, Maltodextrin, Cyclodextrin und Derivate davon, natürliche oder synthetische Aromastoffe und Gemische davon ausgewählt ist, und
(b3) gegebenenfalls 0,05 bis 3 Gewichtsteile eines oberflächenaktiven Bestandteils und
(c) Wasser in einer ausreichenden Menge, um die genannte Viskosität von 0,1-0,3 Pa.s zu erreichen;
(2) die Extrusion des auf diese Weise erhaltenen Gemisches bei Umgebungstemperatur (15-20°C) und die Zerkleinerung des Extrudats zu Tröpfchen mittels Vibrationen bei einer Frequenz von 50 bis 10 000 Hz;
(3) das Einfrieren der auf diese Weise erhaltenen Tröpfchen mittels eines inerten Gases im Gegenstrom, während die Tröpfchen unter Schwerkrafteinwirkung fallen, wobei das Einfrieren in einer Lyophilisationsvorrichtung bis zu einer Temperatur von -30°C bis -50°C fortgesetzt wird;
und
(4) die Beseitigung des Wassers in der Lyophilisationsvorrichtung durch Sublimation.

13. Verfahren nach Anspruch 1, dadurch gekennzeichnet, daß in der Stufe (1) der Wirkstoff aus der Gruppe Paracetamol, Probucol, Piroxicam, Phloroglucinol, Tiadenol, Flerobuterol, Modafinil, Dexfenfluramin, Carbinoxaminmaleat, Loperamid, Lorazepam und Gemische davon ausgewählt wird.
